# EUROPEAN PATENT SPECIFICATION

(11) **EP 1 856 295 B1**
(45) Date of publication and mention of the grant of the patent: **03.04.2013**
(21) Application number: 06748221.6
(22) Date of filing: 16.02.2006
(51) Int. Cl.: C12Q 1/68

(54) **COMPOSITIONS AND METHODS FOR DETECTING VIRAL SPECIFIC NUCLEIC ACIDS IN URINE**
ZUSAMMENSETZUNGEN UND VERFAHREN ZUR DETEKTION VIRALER SPEZIFISCHER NUKLEINSÄUREN IM HARN
COMPOSITIONS ET PROCEDES POUR DETECTER DES ACIDES NUCLEIQUES VIRAUX SPECIFIQUES DANS L'URINE

(30) Priority: 17.02.2005 IT RM20050068; 25.05.2005 US 137935
(43) Date of publication of application: 21.11.2007
(73) Proprietor: TrovaGene, Inc., San Diego, CA 92121 (US)
(72) Inventor: MELKONYAN, Hovsep, Princeton, New Jersey 08540 (US); CANNAS, Angela, I-09031 Arbus (IT); TOMEI, Louis, David, I-00030 Genazzano, Rome (IT); UMANSKY, Samuil, R., Princeton, New Jersey 08540 (US)
(74) Representative: Roques, Sarah Elizabeth
(86) International application number: PCT/US2006/005792
(87) International publication number: WO 2006/089203

(56) References cited:
- WO-A2-98/58086
- US-A- 5 712 385
- US-A1- 2002 119 478
- US-B1- 6 492 144
- LI J J ET AL: "HIV-1 DNA proviral sequences in fresh urine pellets from HIV-1 seropositive persons." LANCET. 30 JUN 1990, vol. 335, no. 8705, 30 June 1990 (1990-06-30), pages 1590-1591, XP009076405 ISSN: 0140-6736
- SU YING-HSIU ET AL: "Human urine contains small, 150 to 250 nucleotide-sized, soluble DNA derived from the circulation and may be useful in the detection of colorectal cancer." THE JOURNAL OF MOLECULAR DIAGNOSTICS : JMD. MAY 2004, vol. 6, no. 2, May 2004 (2004-05), pages 101-107, XP002412388 ISSN: 1525-1578
- LICHTENSTEIN A V ET AL: "Circulating nucleic acids and apoptosis" ANNALS OF THE NEW YORK ACADEMY OF SCIENCES, NEW YORK ACADEMY OF SCIENCES, NEW YORK, NY, US, vol. 945, September 2001 (2001-09), pages 239-249, XP002401431 ISSN: 0077-8923
- BOTEZATU I ET AL: "GENETIC ANALYSIS OF DNA EXCRETED IN URINE: A NEW APPROACH FOR DETECTING SPECIFIC GENOMIC DNA SEQUENCES FROM CELLS DYING IN AN ORGANISM" CLINICAL CHEMISTRY, AMERICAN ASSOCIATION FOR CLINICAL CHEMISTRY, WASHINGTON, DC, US, vol. 8, PART 1, no. 46, August 2000 (2000-08), pages 1078-1084, XP001080049 ISSN: 0009-9147
- SU YING-HSIU ET AL: "Transrenal DNA as a diagnostic tool - Important technical notes" ANNALS OF THE NEW YORK ACADEMY OF SCIENCES NEW YORK ACAD SCIENCES, 2 EAST 63RD ST, NEW YORK, NY 10021 USA SERIES : ANNALS OF THE NEW YORK ACADEMY OF SCIENCES (ISSN 0077-8923(PRINT)), 2004, pages 81-89, XP001248782 & 3RD INTERNATIONAL SYMPOSIUM ON CIRCULATING NUCLEIC ACIDS IN PLASMA/SERUM (CNAPS III) AND SERUM PROTE; SANTA MONICA, CA, USA; NOVEMBER 09 -12, 2003 ISSN: 1-57331-551-6(H) 1-57331-552-4
- ECHAVARRIA M ET AL: "PCR METHOD FOR DETECTION OF ADENOVIRUS IN URINE OF HEALTHY AND HUMAN IMMUNODEFICIENCY VIRUS-INFECTED INDIVIDUALS" JOURNAL OF CLINICAL MICROBIOLOGY, WASHINGTON, DC, US, vol. 36, no. 11, November 1998 (1998-11), pages 3323-3326, XP000957714 ISSN: 0095-1137
- TAMARIT AMPARO ET AL: "Human cytomegalovirus (HCMV)-specific CD4+ T lymphocyte response in AIDS patients with no past or current HCMV disease following HAART." JOURNAL OF CLINICAL VIROLOGY : THE OFFICIAL PUBLICATION OF THE PAN AMERICAN SOCIETY FOR CLINICAL VIROLOGY. APR 2004, vol. 29, no. 4, April 2004 (2004-04), pages 308-314, XP002412389 ISSN: 1386-6532
- JEONG BYUNG-HOON ET AL: "Genotyping of the JC virus in urine samples of healthy Korean individuals." JOURNAL OF MEDICAL VIROLOGY, vol. 72, no. 2, February 2004 (2004-02), pages 281-289, XP002412390 ISSN: 0146-6615
- LO Y M: "Molecular testing of urine: catching DNA on the way out." CLINICAL CHEMISTRY. AUG 2000, vol. 46, no. 8 Pt 1, August 2000 (2000-08), pages 1039-1040, XP002412391 ISSN: 0009-9147

## Description

### BACKGROUND OF THE INVENTION

There are currently three types of *in vitro* diagnostic systems widely used for the detection of pathogens. These are direct culture of the pathogenic agent from the biological sample; immunological assays based on the detection of products or antigens of the infectious agent; and indirect immunological assays that can detect antibodies produced against the infectious agent during infection.

In the first system, the principal disadvantage is that the biological sample must be considered to be at risk for the transmission of the pathogenic agent. In the second and third systems, the disadvantages include sample retrieval that is often invasive and potentially infective sample when collected. In the third system, one major disadvantage is that there is often little possibility of discriminating between past and current infections.

More recently, molecular diagnostic methods have been developed based on the detection of the nucleic acids of the pathogenic agent in the blood or plasma samples, or in the cell cultures, taken from the patient. These assays are generally much more sensitive than the immunological assays. However, they may require the presence of special equipment and qualified personnel. Furthermore, the biological samples — in the case of plasma, blood, or cell cultures — are difficult to store unaltered, except under controlled temperature conditions, and are considered to be biohazardous to personnel who handle them.

Recently, molecular diagnostic methods based on transrenal DNA (Tr-DNA) have been described for monitoring the progress of allogeneic transplants, to diagnose the sex of a fetus, and to detect the presence of tumor markers. (Botezatu et al. Clinical Chemistry 46(8):1078-84 (2000); Su et al. Ann. NY Acad. Sci. 1022:81-89 (2004)) For example, U.S. Patent No. 6,251,638 describes an analytical method for detecting male fetal DNA in the urine of pregnant women. U.S. Patent No. 6,287,820 describes a system aimed at the diagnosis of tumors, particularly of adenocarcinomas of the colon and pancreas. U.S. Patent No. 6,492,144 teaches that the Tr-DNA nucleic-acid analysis method may be used to monitor the progress of allogeneic transplants. The presence of transrenal DNA in urine, in the form of nucleic acid fragments of fewer than 1000 base pairs was also described in Al-Yatama et al. (2001), Prenat Diagn, 21:399-402; and Utting, M., et al. (2002), Clin Cancer Res, 8:35-40. Keiko Koide, et al., Prenat Diagn, 2005; 25: 604-607; Mengjun Wang, et al.., Clinical Chemistry, 2004, 50: 211-213; Y.-H. Su, et al., J. Mol. Diagn., 2004, 6: 101-107.

The presence of transrenal DNA has been explained as being the result of phenomenon of apoptosis. In the process of apoptosis or programmed cell death the nuclear DNA is cleaved into nucleosomes and oligomers, which subsequently, as a part of apoptotic process, are phagocytozed and removed from the organism. (Umansky, S.R., et al. (1982); Biochim. Biophys. Acta, 655:9-17). A portion of this degraded DNA, though, escapes the phagocytosis, and appears in the bloodstream (Lichtenstein, A.V., et al. (2001), Ann NYAcad Sci, 945:239-249), and, as confirmed in the above-referred patents, also in urine.

The presence of viral DNA that originates from sources outside of the urinary tract, in urine had not been described prior to this application. Circulation of viral DNA released from the genome of transfected cell in the plasma has been shown. For example, fragments of Epstein-Barr viral DNA were detected in plasma of patients with nasopharyngeal carcinoma (Chan, K.C., et al. (2002), Cancer Res 63:2028-2032). Also, human papilloma virus (HPV) viral DNA has been detected in the plasma of patients with cervical cancer (Pornthanakasem, W., et al. (2001); BMC Cancer 1:2). However, these viral DNAs were not detected in the urine of the patients.

The instant invention describes a method of detecting the presence of viral pathogens in a subject through the detection of DNA sequences from those pathogens in the urine of the subject.

### SUMMARY OF THE INVENTION

The present disclosure relates to methods for diagnosis and monitoring of viral infections in a subject by detecting and quantifying transrenal viral nucleic acid-specific sequences in urine from the subject. In one embodiment, the nucleic acids are isolated or purified. This purification may be performed using chemical or physical methods. These methods include extraction with organic solvents, filtration, precipitation, absorption on solid matrices having an affinity for the nucleic acids, and chromatography. Solid matrices used in these methods may be constructed from silica-based resins, ion-exchange resins, or hydroxyapatite. In another embodiment, the solid matrix is a silica-based resin, and said isolation or purification is performed in the presence of a chaotropic agent. In another embodiment, one or more agents are added to the urine sample that inhibit the degradation of the nucleic acids. These agents include ion-chelating agents, denaturing agents, and ionic detergents. An example of an ion-chelating agents used in the methods of the invention is EDTA. Examples of denaturing agents used in the methods of the invention are guanidine HCl or guanidine isothiocyanate. Examples of ionic detergents used in the methods of the invention are N-lauryl sarcosine or sodium dodecyl sulfate. In one embodiment, isolated or purified nucleic acids are used for the detection of viral nucleic acids.

In other embodiments, the method also includes the fractionation of the urine sample, for example, through centrifugation or filtration, with the separation of a cell-free fraction from a fraction associated with the cell bodies.

The analysis of the nucleic acids is performed using one of the following techniques: hybridization of the nucleic acids, the cycling probe reaction, a polymerase chain reaction, a nested polymerase chain reaction (PCR), a semi-nested PCR, single-strand conformation polymorphism, a ligase chain reaction, strand displacement amplification, and restriction fragment length polymorphism. In another embodiment, PCR is performed using one or more primers that are specific for the HIV-1 GAG or POL genes. Examples of these primers are described in the specification as SEQ ID NOs:1-11.

In another embodiment, the viral nucleic acids are less than about 1000 bp in size. In a preferred embodiment the viral nucleic acids are between about 100 and about 200 bp in size.

The methods of the invention are applicable to all viral pathogenic agents, including RNA, DNA, episomal, and integrative viruses. They also apply to recombinant viruses, such as the adenoviruses or lentiviruses utilized in gene therapy. In particular, the methods apply to the following viruses: retroviruses, including recombinant and natural HIV-1, HIV-2, variola virus, poliovirus, herpes simplex virus (HSV), Epstein-Barr virus (EBV), hepatitis C virus (HCV), hepatitis B virus (HBV) and adenoviruses (AAV). In some embodiments the viral agents are Epstein-Barr virus (EBV) and HIV-1. In one embodiment, the methods of the invention are performed *in vitro.*

In another embodiment, the invention relates to a method for monitoring a viral infection in a subject, including the steps of quantitating the amount of a transrenal viral nucleic acid in a first urine sample from said subject; quantitating the amount of said transrenal viral nucleic acid in a second urine sample from said subject; and comparing the amount of said transrenal viral nucleic acid in said first and said second urine sample from said subject, thereby monitoring said viral infection in said subject. In another aspect of this embodiment, the method further comprises the step of administering to said subject a compound that reduces or inhibits said viral infection. Optionally, the viral infection is HIV infection and said compound is an anti-viral agent. In another aspect of this embodiment, the quantitating is performed by a means selected from pairing with molecular probes that are specific for those pathogenic agents, hybridization, PCR, nested PCR, semi-nested PCR, SSCP, LCR, and SDA. In another aspect of this embodiment, the method further comprises the step of separating said urine samples into a cell-free fraction containing said transrenal nucleic acids. This separation may be done using centrifugation. The nucleic acids of this embodiment may be between 100 and 200 bp. In another aspect of this embodiment, the subject is at risk for developing a recurring viral infection.

In another of its embodiments, the invention relates to a kit for the detection of viral nucleic acid in urine as disclosed in the appended claims, including: reagents and/or materials for the fractionation and/or extraction of transrenal viral nucleic acids from urine, DNA probe, or pairs of specific oligonucleotides (primers) for at least one viral agent. In one aspect of this embodiment, the probe is a primer for a PCR reaction that is specific for the HIV-1 GAG or POL genes. Examples of these primers are described in the specification as SEQ ID NOs:1-11.

Unless otherwise defined, all technical and scientific terms used herein have the same meaning as commonly understood by one of ordinary skill in the art to which this invention relates. Although methods and materials similar or equivalent to those described herein can be used in the practice or testing of the present invention, suitable methods and materials are described below. In the case of conflict, the present specification, including definitions, will control. In addition, the materials, methods, and examples are illustrative only and not intended to be limiting.

Other features and advantages of the invention will be apparent from the following detailed description and claims.

### BRIEF DESCRIPTION OF THE FIGURES

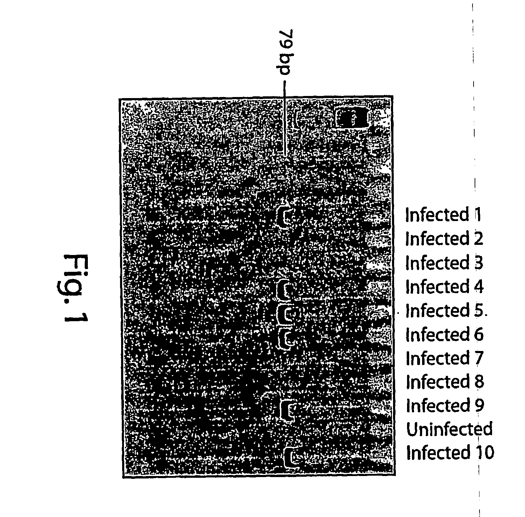

Figure 1 is a photographic image showing the results of gel electrophoresis of the amplification product obtained through nested PCR of the transrenal DNA of non-fractionated urine samples of patients infected with HIV-1 (20 cycles with the 468/602 primer pair [134 bp] followed by 35 cycles with 518/596 [79 bp]). The primers are specific for the HIV-1 GAG region.

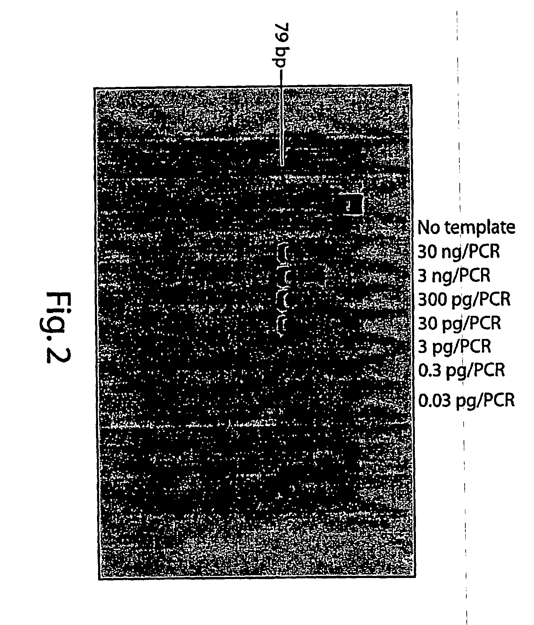

Figure 2 is a photographic image showing the results of gel electrophoresis of the amplification product obtained through nested PCR of the genomic DNA extracted from 8E5 LAV cells (20 cycles with the 468/602 primer pair [134 bp] followed by 35 cycles with 518/596 [79 bp]). The primers are specific for the HIV-1 GAG region.

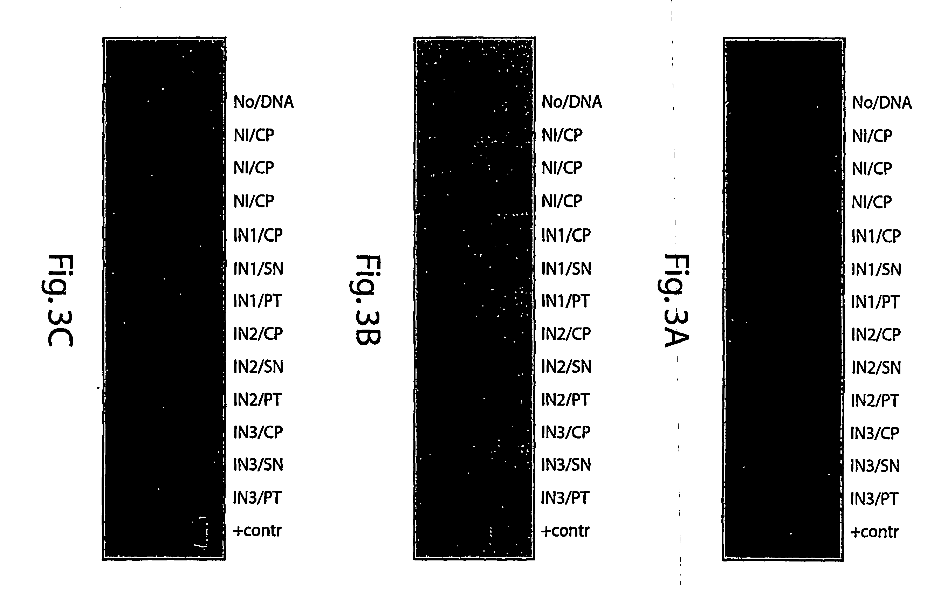

Figure 3 is a photographic image showing the results of gel electrophoresis of the amplification product obtained through nested PCR of the transrenal DNA of urine samples of patients infected with HIV-1 (CP: complete; SN: supernatant; PT: pellet). A-102bp/60bp (specific primers for the HIV-1 POL region); B-317bp/60bp (specific for POL); C-569bp/132bp (specific for TAT); NI: non-infected; IN 1-3: patients infected with HIV.

### DETAILED DESCRIPTION OF THE INVENTION

The present definitions are offered for the purposes of the present invention:
**Amplicon:** A term for any relatively small, DNA fragment that is replicated, *e.g.*, by PCR.
**Amplification:** An increase in the number of copies of a specific DNA fragment can occur *in vivo* or *in vitro.*
**Apoptosis:** Programmed cell death in normally functioning human and animal cells when age or state of cell health and condition dictate. An active process requiring metabolic activity by the dying cell, characterized by cleavage of the DNA into fragments that give a so called laddering pattern of DNA fragments of nucleosomal size and its oligomers.
**Chaotropic:** The property of chemical substances (e.g., ions such as SCN, ClO₄⁻, and guanidine) that disturb the thermodynamic structure of water. It allows less polar and more hydrophobic substances to become more soluble in water. The effect at the biological level is the denaturation of proteins.
**Episome:** A circular DNA molecule of that can replicate independently of the cellular chromosome or integrate and replicate as part of the chromosome.
**Gene:** DNA fragment that contains sequences necessary to code for an mRNA, and to control the expression of these sequences.
**Genome:** The total set of genes of an organism enclosed, among the eukaryotes, in chromosomal structures.
**Cyclic Probe Reaction:** CPT reactions are performed at a constant specific temperature, which allows hybridization of the chimeric probe with its complementary single-stranded target DNA. Within the resulting target-probe duplex, RNase H recognizes the DNA-RNA hybrid and specifically cleaves the RNA portion of the probe. The cleaved fragments are not stable at the reaction temperature and disassociate from the target. The target is then free to hybridize with another probe molecule, and the cycle is repeated. The probe fragments accumulate, serving as a basis for the detection of target. Over time, the accumulation of cleaved probe fragments follows linear kinetics and therefore the amount of target can be quantified.
**Hybridization:** A widely used technique that exploits the ability of complementary sequences in single-stranded DNAs or RNAs to pair with each other to form a double helix. Hybridization can take place between two complimentary DNA sequences, between a single-stranded DNA and a complementary RNA, or between two RNA sequences. The technique is used to detect and isolate specific sequences, measure homology, or define other characteristics of one or both strands.
**Infection:** Invasion and multiplication of microorganisms in body tissues, which may be clinically unapparent or result in local cellular injury due to competitive metabolism, toxins, intracellular replication or antigen antibody response.
**Ligase Chain Reaction:** A method of DNA amplification similar to PCR. LCR differs from PCR because it amplifies the probe molecule rather than producing amplicon through polymerization of nucleotides. Two probes are used per each DNA strand and are ligated together to form a single probe. LCR uses both a DNA polymerase enzyme and a DNA ligase enzyme to drive the reaction. Like PCR, LCR requires a thermal cycler to drive the reaction and each cycle results in a doubling of the target nucleic acid molecule. LCR can have greater specificity than PCR.
**Nested PCR:** A second PCR that is performed on the product of an earlier PCR using primer, which are internal to the originals. This significantly improves the sensitivity and specificity of the PCR.
**Nested primer:** A selected primer internal to an amplicon obtained with a first PCR cycle. The amplification process that uses at least one nested primer improves specificity, because the non-specific products of the first cycle are not amplified in the second cycle.
**Nucleic Acid:** Linear polymers of nucleotides, linked by 3', 5' phosphodiester linkages. In DNA, deoxyribonucleic acid, the sugar group is deoxyribose and the bases of the nucleotides adenine, guanine, thymine and cytosine. RNA, ribonucleic acid, has ribose as the sugar and uracil replaces thymine. DNA functions as a stable repository of genetic information in the form of base sequence. RNA has a similar function in some viruses but more usually serves as an informational intermediate (mRNA), a transporter of amino acids (tRNA), in a structural capacity or, in some newly discovered instances, as an enzyme.
**Oligonucleotide/Polynucleotide:** Linear sequence of two or more nucleotides joined by phosphodiester bonds. Above a length of about 20 nucleotides the term "polynucleotide" is generally used.
**Pathogenic agent:** A pathogen is a biological agent that can cause disease to its host. A synonym of pathogen is "infectious agent". The term "pathogen" is most often used for agents that disrupt the normal physiology of a multicellular organism.
**Pellet:** Sediment, when cells are present, usually includes the cell fraction, or that can be obtained by centrifuging a biological sample.
**Polymerase:** Enzyme utilized in the amplification of nucleic acids. The term includes all of the variants of DNA polymerases.
**Primer:** Short pre-existing polynucleotide chain to which new deoxyribonucleotides can be added by DNA polymerase.
**PCR:** Polymerase Chain Reaction involving two synthetic oligonucleotide primers, which are complementary to two regions of the target DNA (one for each strand) to be amplified, are added to the target DNA (that need not be pure), in the presence of excess deoxynucleotides and Taq polymerase, a heat stable DNA polymerase. In a series (typically 30) of temperature cycles, the target DNA is repeatedly denatured (around 90 °C), annealed to the primers (typically at 50-60 °C) and a daughter strand extended from the primers (72 °C). As the daughter strands themselves act as templates for subsequent cycles, DNA fragments matching both primers are amplified exponentially, rather than linearly.
**Probe:** General term for a fragment of DNA or RNA corresponding to a gene or sequence of interest that has been labelled either radioactively or with some other detectable molecule, such as biotin, digoxygenin or fluorescein.
**Purification / Decontamination / Sterilization:** Refers to a process for removing contaminants from a sample, where the result is a sample containing 60%, preferably 75%, and even more preferably 90% of the material toward which the purification procedure is directed.
**Restriction Fragment Length Polymorphism (RFLP):** A method that allows genetic relationship established by comparing the characteristic polymorphic patterns that are obtained when certain regions of genomic DNA are amplified (typically by PCR) and cut with certain restriction enzymes. Variations in such patterns are generated by mutations that create or abolish recognition sites for these enzymes
**Sample:** The term is broadly interpreted and includes any form that contains nucleic acids (DNA or RNA) in solution or attached to a solid substrate, where the definition of "nucleic acids" includes genomic DNA (for example, when it is attached to a solid substrate, such as in the Southern Blot or in solution), cDNA, and other forms.

Combinations of two nucleic-acid sequences through hybridization are formed thanks to the hydrogen bonds between G and C or A and T bases or analogs of these bases. These combinations are complementary, and the DNA helixes are anti-parallel. This hybridization combination can be created with one sequence (or helix) in a solution and the other attached to a solid phase (such as, for example, in the FISH [fluorescent *in situ* hybridization] method), or else with both of the sequences in solution.
**Single-Strand Conformation Polymorphism (SSCP):** SSCP is the electrophoretic separation of single-stranded nucleic acids based on subtle differences in sequence (often a single base pair) that results in a different secondary structure and a measurable difference in mobility through a gel.
**Strand Displacement Amplification (STA):** STA is an isothermal, in vitro nucleic acid amplification technique based upon the ability of HincII to nick the unmodified strand of a hemiphosphorothioate form of its recognition site, and the ability of exonuclease deficient Klenow fragment of DNA Polymerase (exo- klenow) to extend the 3'-end at the nick and displace the downstream DNA strand. Exponential amplification results from coupling sense and antisense reactions in which strands displaced from a sense reaction serve as target for an antisense reaction and vice versa.
**Target sequence:** Nucleic-acid sequence that should be analyzed through hybridization, amplification, or other methods or combinations of methods.
**Tm (melting temperature):** Temperature at which a specific double-helix DNA population dissociates into single-strand polymers. The formula for calculating this temperature for polynucleotide fragments is well known in the art: Tm = 81.5 + 0.41 (% G+C) (Anderson & Young, "Quantitative Filter Hybridization," in Nucleic Acid Hybridization [1985]). For oligonucleotides with fewer than 40 base pairs, a simplified formula can be used: Tm = 3°C x (G +C)+2x(A+T).
**Tr-DNA/RNA:** Transrenal DNA/RNA, or DNA/RNA present in urine after having been passed through the kidney barrier.
**Urinary tract:** Includes the organs and ducts that participate in the elimination of urine from the body.

### Urinary nucleic acids in viral pathogen infections

The present invention is based on the discovery that following a viral infection, the nucleic acids of the virus(es) or of viral origin are cleaved into relatively short fragments which are found in the urine. Many of these pathogen specific nucleic acids cross the transrenal barrier (these nucleic acids are generally termed TrNA, or TrDNA or TrRNA) and can be detected in urine as cell-free low-molecular-weight fragments (whose length is less than 1000 nucleotides, but are preferably less than 500 bp in length, and more preferably shorter than 250-300 bp in length or shorter than 250 bp in length) through molecular methods. These transrenal nucleic acids are derived from viruses which are located outside of the urinary tract of a subject. As used herein, the term "viral nucleic acid" encompasses nucleic acids of viral origin. Other virus specific nucleic acids may be shed by virus or cells that are within the kidney, and thus do not have to cross the transrenal barrier in order to be detected in the urine. Further, some virus specific nucleic acids may be found in the urine through other mechanisms besides crossing the transrenal barrier or being generated by virus in the kidney.

The presence of transrenal nucleic acids (Tr-NA) in urine was detected previously only in hosts who had undergone heterologous tissue or organ transplants, in the case of women pregnant with male fetuses, and in the case of tumors characterized by specific marker genes. The presence oftransrenal nucleic acids of viral origin in the case of viral infections according to the present invention is also, and preferably, detected in the case of non- urinary-tract infections, even in the absence of hematuria or of pathologies that lead to the rupture, or that alter the normal integrity, of the renal barrier.

Transrenal nucleic acids (Tr-NA) of viral origin are not associated with, and are not derived from, the genome of viruses that are lost or released in the urinary tract and that are found in urine. Instead, transrenal nucleic acids are filtered by the glomerular-renal filtration mechanism. Thus, the dimensions of the transrenal nucleic-acid fragments are generally smaller than about 1000 base pairs, e.g., smaller than about 500, smaller than about 300, smaller than about 250, or between about 100 and about 200 base pairs, as opposed to other situations in which DNA usually has a high molecular weight and a length in excess of 1000 bases or base pairs.

Therefore, in the present invention, the transrenal nucleic acid (TrNA) of viral origin is generally not found in the urine sediment, but in the soluble fraction, although traces of TrNA can co-sediment with the cells during centrifuging.

The discovery confirms the presence of urinary nucleic acids or transrenal nucleic acids derived from pathogenic viruses in urine, and therefore is applicable to the diagnosis of all infectious diseases caused by viral pathogens.

Therefore, in embodiments, the invention relates to methods for diagnosis or monitoring of viral infection by determining the presence of viral nucleic acids, preferably viral DNA or RNA of viral origin, in a urine sample. The methods includes the step of determining the presence of transrenal viral nucleic acids using methods generally used in laboratory practice such as hybridization, PCR, nested PCR, semi-nested PCR, SSCP, LCR, and SDA.

In certain embodiments, the methods according to the invention include an initial treatment of the urine sample prior to the determination of the presence of transrenal viral nucleic acids. In an embodiment, the invention includes the pretreatment of the urine sample with an agent that inhibits the degradation of the DNA or RNA. These agents include the enzymatic inhibitors, such as chelating agents, detergents, or denaturing agents, DNase or RNase inhibitors, which are preferably selected from the group consisting of EDTA, guanidine HCl, guanidine isothiocyanate, N-lauryl sarcosine, and sodium dodecyl sulfate.

In another embodiment, the determination of the presence oftransrenal viral nucleic acids optionally is preceded by centrifugation or filtration of the urine sample in order to separate the cellular fraction of the urine from the cell-free low-molecular-weight nucleic acids (DNA/RNA). However, the urine sample may also be utilized without fractionation. Centrifugation is preferably performed at a speed between 2500g and 4500g, and more preferably between 3000g and 4000g. Filtration is preferred to carry out through a filter with pore size between 0.1 and 5.0 µm, more preferably with pore size between 0.2 and 1.0 µm and even more preferably 0.45 and 0.8 µm. Equivalent methods for separating the soluble fraction from the cellular fraction may also be used.

The optional isolation or purification and quantification of the transrenal nucleic acids are achieved through the use of chemical or physical methods that are already known in the art. It includes at least one purification step, using methods selected from among extraction with organic solvents, filtration, precipitation, absorption on solid matrices (e.g., via ion exchange), affinity chromatography or else molecular exclusion chromatography or combinations of these methods.

However, the purification method must be appropriate for the isolation of DNA (single- or double-helix) that are less than 1000 nucleotides in length, with a corresponding molecular weight, assuming, as the average molecular weight, that of a nucleotide having a value of 330 Daltons. In some embodiments, the purification is specific for fragments that are smaller than 500 nucleotides (nt) in length, with a corresponding molecular weight, such as fragments whose lengths are less than 300 nt, fragments less than 250 nt in length, or fragment whose lengths are between 100 and 200 base pairs of nucleic acids (nt).

The isolation and/or purification of the transrenal nucleic acids is achieved through the use of chemical or physical methods that are already known in the art. It includes one or more purification steps using methods selected from among extraction with organic solvents, filtration, precipitation, absorption on solid matrices (e.g., silica resin, hydroxyapatite or ion exchange), affinity chromatography (e.g., via sequence specific capture or nucleic acid specific ligands), or else molecular exclusion chromatography. However, the purification method must be appropriate for the isolation of DNA (single- or double-strand) whose dimensions are smaller than 1000 nucleotide pairs. Even more preferably, the purification is specific for fragments that are smaller than 500 nucleotides, and even more preferably, fragments whose length are less than 300 or 250 base pairs, or that are between 100 and 200 bases or base pairs. The purification preferably takes place on a matrix consisting but not limited to a silica resin.

In one preferred embodiment, the DNA isolation method is implemented by pretreating the urine sample with a denaturing agent, as described above, e.g., urea, guanidine HCl, or guanidine isothiocyanate, at room temperature. Guanidine isothiocyanate is preferably utilized. The sample is then passed through a solid phase, preferably a matrix consisting of a silica resin that, in the presence of chaotropic salts (guanidine isothiocyanate), binds the nucleic acids. The sample is then collected or eluted in a buffer, such as Tris-EDTA (Tris 10 mM, EDTA 1 mM), or in water.

In another preferred embodiment, the characterization and the determination of the presence of transrenal viral NA are performed through a technique selected from the group consisting of: hybridization of the nucleic acids, a cycling probe reaction (F. Bekkaoui et al., in BioTechniques 20:240-248 [1996]), a polymerase chain reaction (PCR Protocols: A Guide to Methods and Applications, by M. Innis et al.; Elsevier Publications, 1990), a nested polymerase chain reaction, single-strand conformation polymorphism, a ligase chain reaction (LCR) (F. Barany, in PNAS USA, 88:189-93 [1991]), strand displacement amplification (SDA) (G.K. Terrance Walker, et al., in Nucleic Acid Res, 22:2670-77 [1994], and restriction fragments length polymorphism (RFLP). A technician in the field might also use combinations of these methods, e.g., PCR-Restriction Length Polymorphism, in which the nucleic acids are amplified, and then divided into aliquots and digested with restriction enzymes, and then separated via electrophoresis.

Polymerase chain reaction (PCR) is the preferred method for the detection and/or quantitative analysis of nucleic acids. Yet more preferred is the nested PCR method, as defmed above, or the semi-nested PCR method, in which only one of the two primers is internal to the amplicon.

The advantage of the method is linked primarily to the ease of collecting the biological samples; to the fact that the transrenal nucleic acids are not infectious; and to the sensitivity of the molecular diagnostic method that can be applied to the nucleic acids, even in the form of fragments.

The diagnostic method is applicable to all viral pathogenic agents, including RNA, DNA, episomal, or integrated viruses. It also applies to recombinant viruses, such as the adenoviruses or lentiviruses utilized in gene therapy. In particular, the method preferably applies to the following viruses: recombinant and natural HIV-1, HIV-2, variola virus, poliovirus, herpes simplex virus (HSV), Epstein-Barr virus (EBV), hepatitis C virus (HCV), hepatitis B virus (HBV) and adenoviruses (AAV).

The method is preferably applied to the HIV-1 virus, with the selection of probes or primer oligonucleotides in the GAG, POL, or TAT region for the detection. Particularly preferred pairs of primers are the ones consisting of the specific sequences for the HIV GAG region, and preferably the ones corresponding to the IDN 1-4 sequence, and the ones that are specific for the HIV-1 POL region, preferably the ones corresponding to the IDN 5-7 region, when the detection is performed via a polymerase chain reaction (PCR), and, in particular, via nested (GAG) or semi-nested (POL) PCR.

The invention relates to a kit for the detection and monitoring of transrenal viral NA in urine as disclosed in the appended claims, including: reagents and/or materials for the separation and/or purification of transrenal DNA from a urine sample, DNA probes, or pairs of specific oligonucleotides (primers) for at least one viral agent. Reaction tubes, agents for the pretreatment of the sample, enzymes for labeling the probe, and enzymes for the amplification of the DNA may optionally be present.

In a preferred embodiment, the kit includes pairs of oligonucleotide primers that are specific for recombinant and natural HIV-1, HIV-2, variola virus, poliovirus, herpes simplex virus (HSV), Epstein-Barr virus (EBV), hepatitis C virus (HCV), hepatitis B virus (HBV), adenoviruses (AAV); or, yet more preferably, primers that are selected from the group consisting of the sequences listed below, and specific reagents for the polymerization chain reaction, preferably in nested or semi-nested form.

### Methods for the Amplification and Detection of Urinary Nucleic Acids

The term nucleic acid refers to an oligonucleotide, nucleotide, polynucleotide, or fragments/parts thereof and to DNA or RNA of natural (*e.g.*, genomic) or synthetic origin. It may have a double or single helix, and may also represent the sense or antisense direction of a sequence. Parallel helix (5'→3'); antiparallel helix (3'→5'). The terms oligonucleotide, polynucleotide and nucleic-acid polymer are equivalent, and are understood as referring to a molecule consisting of more than two deoxyribonucleic or ribonucleic acid bases. The number of nucleotides (bases) and the length of the oligonucleotide fragment may vary. They may be synthesized in different ways. The sequences are traditionally defined as starting with 5' and ending with a 3'. These numbers indicate the direction of the sequence.

DNA isolated from the urine of a subject may then be amplified in order to be detected. Amplification methods include polymerase chain reaction (PCR), nested PCR, semi-nested PCR, Single-Strand Conformation Polymorphism analysis (SSCP), ligase chain reaction (LCR) and strand displacement amplification (SDA). Detection oftransrenal DNAs is also performed through hybridization of at least one labeled primer.

Hybridization is a method that allows two nucleic-acid sequences to recognize each other as complementary and to join together (annealing). Complementarity / Complementary sequences are sequences of polynucleotides that interact with each other, depending on the interaction between the bases. For example, the AGTC sequence is complementary to TCAG according to standard Watson Crick base pairing. However, other combinations such as Hoogstein base pairing are well known to those having ordinary skill in the art. It is possible to have a fully or partially complementary sequence, and this is what determines the efficiency or attractive force between the two sequences. Average complementarity would prevent a strong complementarity from hybridizing, under conditions that would allow it to remain attached.

The ability of nucleic sequences to hybridize is a well-known phenomenon. The first hybridization method was described in Marmur & Lane, PNAS USA, 46:453 (1960) and 461 (1960), but since then has been perfected as a technique in molecular biology. Today, the term "hybridization" includes, among others, slot/dot and blot hybridization. The conditions that allow nucleotide sequences to recognize each other (hybridization) can be modified in such a way as to produce complete hybridization (complementarity with high specificity) or partial hybridization (complementarity with average specificity). In the present application, whenever the term "hybridization" is used, the conditions should be understood as referring to those that allow average or high complementarity. The technician in the field can calculate how many artificial sequences are needed to encourage hybridization between two complementary sequences in the opposite direction, known as antiparallel association.

A probe is an oligonucleotide that can be produced artificially or naturally, and that forms a combination with another nucleic-acid sequence. The probes are useful in discovering specific sequences in a sample containing unknown DNA. In this patent, all of the probes can be bound to a signaling molecule (or reporter). The reporter molecule makes it possible to detect the probe (for example, through enzymatic reactions (*e.g.*, ELISA (Enzyme-Linked Immunosorbent Assay)), radioactivity, fluorescence, or other systems).

Polymerase chain reaction (PCR) is a method of amplification of a DNA sequence using complementary primers and a heat sensitive polymerase. One class of enzymes utilized in the amplification of specific nucleic acids are DNA polymerases referred to as Taq (*Thermus aquaticus*) polymerases. Primers are oligonucleotides from which, under proper conditions, the synthesis of a polynucleotide sequence can be initiated. A primer may exist naturally (for example, in an enzymatic digestion of a polynucleotide), or may be obtained through chemical synthesis. The product amplified in PCR is often referred to as an amplicon.

Nested PCR is a second PCR which is performed on the product of an earlier PCR using a second set of primers which are internal to the first set of primers, referred to as nested primers. This significantly improves the sensitivity and specificity of the PCR. Nested primers are primers internal to an amplicon obtained with a first PCR cycle. The amplification process that uses at least one nested primer improves specificity, because the non-specific products of the first cycle are not amplified in the second cycle, because they lack the sequence that corresponds to the nested primer. Semi-nested PCR is a second PCR which uses one new primer and one of the original primers. This process also improves specificity.

Ligase Chain Reaction (LCR) is a method of DNA amplification similar to PCR. LCR differs from PCR because it amplifies the probe molecule rather than producing an amplicon through polymerization of nucleotides. Two probes are used per each DNA strand and are ligated together to form a single probe. LCR uses both a DNA polymerase enzyme and a DNA ligase enzyme to drive the reaction. Like PCR, LCR requires a thermal cycler to drive the reaction and each cycle results in a doubling of the target nucleic acid molecule. LCR can have greater specificity than PCR.

In Single-Strand Conformation Polymorphism (SSCP) analysis a small PCR product (amplicon) is denatured and electrophoresed through a non-denaturing polyacrylamide gel. Thus, as the PCR product moves into and through the gel (and away from the denaturant), it will regain secondary structure that is sequence dependent (similar to RNA secondary structure). The mobility of the single-stranded PCR products will depend upon their secondary structure. Therefore, PCR products that contain substitutional sequence differences as well as insertions and deletions will have different mobilities.

Strand displacement amplification (SDA) is an isothermal nucleic acid amplification method based on the primer-directed nicking activity of a restriction enzyme and the strand displacement activity of an exonuclease-deficient polymerase.

The terms purification or isolation refers to a process for removing contaminants from a sample, where the result is a sample containing 50%, 60%, 75%, 90% or over 90% of the material toward which the purification procedure is directed.

For stringent temperature conditions in the case of nucleic-acid hybridization, these terms usually refer to a variable temperature between a maximum, for a nucleic acid, represented by Tm less 5 °C, and a minimum represented by Tm less 25 °C. The technique used in the field utilizes stringent temperature conditions, in combination with other parameters (*e.g.*, saline concentration), to distinguish sequences with a quasi-exact homology.

Stringent conditions are known to those skilled in the art and can be found in Ausubel, et al., (eds.), CURRENT PROTOCOLS IN MOLECULAR BIOLOGY, John Wiley & Sons, N.Y. (1989), 6.3.1-6.3.6. Preferably, the conditions are such that sequences at least about 65%, 70%, 75%, 85%, 90%, 95%, 98%, or 99% homologous to each other typically remain hybridized to each other. A non-limiting example of stringent hybridization conditions are hybridization in a high salt buffer comprising 6X SSC, 50 mM Tris-HCl (pH 7.5), 1 mM EDTA, 0.02% PVP, 0.02% Ficoll, 0.02% BSA, and 500 mg/ml denatured salmon sperm DNA at 65°C, followed by one or more washes in 0.2X SSC, 0.01% BSA at 50°C.

In a second embodiment, a nucleic acid sequence that is hybridizable to the nucleic acid molecule comprising the nucleotide sequence, or fragments, analogs or derivatives thereof, under conditions of moderate stringency is provided. A non-limiting example of moderate stringency hybridization conditions are hybridization in 6X SSC, 5X Reinhardt's solution, 0.5% SDS and 100 mg/ml denatured salmon sperm DNA at 55 °C, followed by one or more washes in 1X SSC, 0.1% SDS at 37 °C. Other conditions of moderate stringency that may be used are well-known within the art. *See, e.g.,* Ausubel, et al. (eds.), 1993, CURRENT PROTOCOLS IN MOLECULAR BIOLOGY, John Wiley & Sons, NY, and Krieger, 1990; GENE TRANSFER AND EXPRESSION, A LABORATORY MANUAL, Stockton Press, NY.

In a third embodiment, a nucleic acid that is hybridizable to the nucleic acid molecule, or fragments, analogs or derivatives thereof, under conditions of low stringency, is provided. A non-limiting example of low stringency hybridization conditions are hybridization in 35% formamide, 5X SSC, 50 mM Tris-HCl (pH 7.5), 5 mM EDTA, 0.02% PVP, 0.02% Ficoll, 0.2% BSA, 100 mg/ml denatured salmon sperm DNA, 10% (wt/vol) dextran sulfate at 40°C, followed by one or more washes in 2X SSC, 25 mM Tris-HCl (pH 7.4), 5 mM EDTA, and 0.1% SDS at 50°C. Other conditions of low stringency that may be used are well known in the art (*e.g.*, as employed for cross-species hybridizations). *See, e.g.,* Ausubel, et al. (eds.), 1993, CURRENT PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY; Shilo and Weinberg, 1981. Proc Natl Acad Sci USA 78: 6789-6792.

The invention also provides a kit for detecting and/or genotyping a viral nucleic acid in a urine sample from a subject in need thereof, comprising at least one forward primer selected from SEQ ID NOs: 1, 3, 5, 9 and 11 and at least one reverse primer selected from SEQ ID NOs: 2, 4, 6, 7, 8 and 10 either in the same or separate packaging, and instructions for its use. In one embodiment, this viral nucleic acid is derived from HIV.

The invention will be further described in the following examples, which do not limit the scope of the invention described in the claims.

### EXAMPLES

The technician in the field may modify all of the methodology described herein with no change in the basic principal idea.

### Example 1. Stabilization and preparation of the samples.

All of the steps of the preparation of the urine samples and of the analysis of the transrenal DNA were performed at room temperature. Briefly, approximately 50-60 ml of urine samples were collected from each patient participating in the study. Within 30 minutes after collection, a solution consisting of 0.5M EDTA and 0.5M Tris-HCl, at a pH ranging from 8.0 to 8.5, was added at a final concentration of 10 mM in order to inhibit the nucleases that might be present in urine samples. At least three nucleolytic enzymes were identified in urine, DNase I, DNase II and phosphodiesterase. EDTA inhibits the activity of DNase I and phosphodiesterase by chelating divalent metals such as Mg²⁺, Ca²⁺ and/or others that are required for their activity. DNase II is maximally active in acidic environment. Supplementing the urine with Tris-HCl pH 8.0 buffer increases the pH of urine thereby inhibiting the activity of DNase II.

Higher amounts of urine may be taken per subject, *e.g.* 100-1000 ml. These larger samples may be concentrated and used to create higher concentrations of very dilute viral nucleic acids found in urine.

The stabilized urine samples can be stored, in aliquots of 5 ml, at -80°C. Optionally, 25 mL of saline solution is frozen to act as a control.

Other methods for the preparation of urine samples and for the extraction of the DNA are described in PCT patent No. WO 98/54364.

### Example 2. Urine fractionation.

For Tr-DNA based applications, that involve quantitative Tr-DNA analysis, urine fractionation is often required. It reduces the impact of nucleoproteins from cells present in urine on accurate quantitation of Tr-DNA/protein complex, total Tr-DNA, or specific genetic markers. In our experiments we have employed two procedures for urine fractionation, centrifugation and filtration. Both were carried out immediately after urine collection and, before supplementing the specimen with EDTA-Tris stabilizing mixture. Urine may be kept in this condition as long as urine cells are intact and their constituents do not leak into urine and contaminate Tr-DNA by cellular DNA.

For filtration one can use filters with pore size ranging from 0.1 to 5 µm with reduced nucleic acid and protein binding capacity. The choice of the filter depends on target under analysis. In our experiments we used luer-lock 0.45 µm Filter Units (Cat. No. SLHV033RS) or 0.45 µm 150 ml Filter Unit STERICAP (Cat. No. SCHVU01RE) both from Millipore. After filtration the samples were supplemented with EDTA-Tris conservation solution and taken into downstream processes of DNA extraction or aliquoted and stored frozen at -80 °C. Urine cellular fraction can be harvested by extracting it from the filter by applying a solution (high concentrations of salts or chaotropic agents) that is known to dissolve cells.

Urine fractionation by centrifugation in our experiments was carried immediately after sample collection, before addition of preservation mixture. Centrifugation was performed at room temperature at RCF ranging between 3500 and 4000xg for approximately 15 min. Supernatant was carefully collected and manipulated per the procedure described above for urine filtration. The pellet consisting mainly of cells and cellular debris was resuspended in physiological solution and stored at -80 °C.

### Example 3. Extraction and purification of the nucleic acids from urine.

In our experiment we used silica based DNA extraction protocol in two different formats. One was vacuum driven and the second, centrifugation.

### Vacuum Based Procedure

To the urine sample 6 M Guanidine Thiocyanate (GITC) (AMRESCO, Cat. No.0380) was added to final concentration not less than 3 M. This solution was mixed vigorously and supplemented with 0.25 ml of Wizard silica suspension (PROMEGA, Cat. No. A7141) per 10 ml of initial urine volume. To capture DNA the mixture was continuously rotated for 1 hour at room temperature. Resin with captured DNA was collected by loading the mixture on a minicolumn (PROMEGA, Cat. No. A7211) syringe assembly attached to a vacuum line. Resin was collected by applying vacuum, washed twice with 5 ml of 3M GITC solution. The resin was further washed twice by a solution consisting of 80% ethanol and 50 mM NaCl. Then the minicolumn was detached and the resin was additionally washed twice with 96% ethanol by centrifugation on a benchtop microcentrifuge in 1.5 ml tubes. DNA was eluted by brief centrifugation with hot water in a volume of not more than 1/20 of urine initial volume.

### Centrifugation Based Procedure

To the urine sample 6 M Guanidine Thiocyanate (GITC) (AMRESCO, Cat. No.0380) was added to final concentration not less than 3 M. This solution was mixed vigorously and supplemented with 0.25 ml of Wizard silica suspension (PROMEGA, Cat. No. A7141) per 10 ml of initial urine volume. To capture the DNA, the mixture was continuously rotated for 1 hour at room temperature. Resin with captured DNA was collected by centrifugation at approximately 4000xg for about 5 min at room temperature. Pelleted resin was washed once with GITC solution and once with wash buffer, then was transferred into a mini-column and washed one additional time with 96% ethanol. DNA was eluted with hot water into a microcentrifuge tube by brief centrifugation. DNA was eluted by brief centrifugation with hot water in a volume of not more than 1/20 of urine initial volume.

### Example 4. Design of PCR primers

The primers for analysis of Tr-DNA based on the use of PCR were selected for two different sizes of the target fragment, *i.e.*, one in the range from 60 to 120 bp and the other in the range from 250 to 400 bp. All of the primers were also compared against the complete human genome sequence. The primers were designed using the FastPCR software package (biocenter.helsinki.fi/bi/bare-1_html/oligos). The primers for the nested-PCR analysis were selected using the Primer 3 package, which is available at the frodo.wi.mit.edu/cg-i-bin/primer3/primer3_www.cgi site, in such a way that the melting temperature of the internal, nested primers was not lower than that of the external primers.

The HIV primers were selected for recognition of all 9 of the Type M HIV subtypes. In accordance with the recently revised nomenclature (*i.e.*, the 1999 Nomenclature Proposal: [hiv.lan1.gov/content/hiv-db/HTML/reviews/ nomenclature/Nomen]), the HIV-1 M group subtypes are represented by phylogenetically associated groups of HIV-1 sequences. They are designated as A1, A2, B, C, D, F1, F2, G, H, J, and K. The M group contains viruses that represent approximately 95% of all of the cases of HIV in Europe. Complete genomic sequences of HIV-1 M group subtypes were obtained from the database at the Los Alamos National Laboratory (New Mexico, hiv.lanl.gov). The multiple alignments and the creation of consensus sequences of the various subtypes were performed through the use of ClustalX algorithm included in the BioEdit package (mbio.ncsu.eduBioEdit/bioedit.html).
The following primers were selected:
GAG primers for nested PCR.
   External:
   GAG 468-F (SEQ ID NO: 1): TGGGTAAAAGTAATAGAGGAGAAGGC;
   GAG 602-R (SEQ ID NO: 2): AACATTTGCATGGCTGCTT.
   Product: 134 bp.
   Internal:
   GAG 518-F (SEQ ID NO: 3): CAGCATTATCAGAAGGAGCCACC;
   GAG 596-R (SEQ ID NO: 4): TGCATGGCTGCTTGATGTCC.
   Product: 79 bp.
   POL primers for semi-nested-PCR, short amplicon
   External primers:
   POL 4368-F (SEQ ID NO: 5): GGRGAAGCCWTGCATGGAC;
   POL 4468-R (SEQ ID NO: 6): GCTACATGRACTGCTACCAG.
   Product: 102 bp.
   Internal primers:
   POL 4368-F (SEQ ID NO: 5): GGRGAAGCCWTGCATGGAC;
   POL 4427-Rn (SEQ ID NO: 7): TGTRCAATCTARTTGCCATATYCCTGG.
   Product: 60 bp.
POL primers for semi-nested-PCR, long amplicon
   External primers:
   POL 4368-F (SEQ ID NO: 5): GGRGAAGCCWTGCATGGAC
   POL 4678-R (SEQ ID NO: 8): ACTCCYTGRCTTTGGGGATTG
   Product: 311 bp.
   Internal primers (nested):
   POL 4368-F (SEQ ID NO: 5): GGRGAAGCCWTGCATGGAC
   POL 4427-Rn (SEQ ID NO: 7): TGTRCAATCTARTTGCCATATYCCTGG.
   Product: 60 bp.
TAT primers for semi-nested PCR (long amplicon)
   External primers:
   TAT 5955-F (SEQ ID NO: 9): GCTTAGGCATYTCCTATGGCAG
   TAT 6462-R (SEQ ID NO: 10): TGGGGTCTGTKGGTACACAGG.
   Product: 569 bp.
   Internal primers:
   TAT 6330-Fn (SEQ ID NO: 11): CWGTHTAYTATGGRGTACCTGTGTGG
   TAT 6462-R (SEQ ID NO: 10): TGGGGTCTGTKGGTACACAGG.
   Product: 132 bp.

### Example 5. Diagnosis of HIV-1 infection based on TrDNA.

The DNA was isolated from the urine samples of 10 patients infected with HIV, as described in the preceding examples. These patients were clinically diagnosed as being infected with HIV through the use of standard clinical molecular tests to detect the presence of antibodies that are specific for the HIV virus.

Figure 1 shows the electrophoresis of the HIV- DNA fragments, as amplified via nested PCR, with the above indicated two pairs of GAG-specific primers, in accordance with the method described in the invention. A band whose dimensions correspond to the ones that were expected as the result of the nested PCR amplification - as performed with the use of the 468/602 pair of primers in the first amplification, followed by the 518/596 pair (79 bp) - was observed in the urine of 8 of the 10 patients and in the positive controls, but not in the negative controls.

Table 1 indicates the viral load of each of the patients who were analyzed, from which it can be inferred that in the case of the samples (numbers 2 and 3) that were negative upon amplification, the viral load was lower than 50 copies of the virus per ml of plasma.

**Table 1. Viral load of the HIV-infected patients.**

| **Patient No.** | **Infection** | **HIV load** |
|---|---|---|
| 1 | HIV | 120,000 |
| 2 | HIV | <50 |
| 3 | HIV | <50 |
| 4 | HIV | 867 |
| 5 | HIV | 88,000 |
| 6 | HIV | 8,370 |
| 7 | HIV | 32,046 |
| 8 | HIV+HCV | 500,000 |
| 9 | HIV | 15,822 |
| 10 | HIV+HCV | <50 |

The viral load was determined by measuring the quantity of the HIV-specific RNA via RT-PCR in the plasma.
The apparent sensitivity of the nested PCR was evaluated by amplifying, with the same pair of primers, an 8E5 LAV cell line genomic DNA carrying a single integrated copy of the HIV genome. The sensitivity was determined to be 5 genome equivalents.

### Sequence summary list

GAG 468-F (SEQ ID NO: 1): TGGGTAAAAGTAATAGAGGAGAAGGC;
GAG 602-R (SEQ ID NO:2): AACATTTGCATGGCTGCTT;
GAG 518-F (SEQ ID NO:3): CAGCATTATCAGAAGGAGCCACC;
GAG 596-R (SEQ ID NO:4): TGCATGGCTGCTTGATGTCC.
POL 4368-F (SEQ ID NO:5): GGR*GAAGCCW*TGCATGGAC;
POL 4468-R (SEQ ID NO:6): GCTACATGR*ACTGCTACCAG;
POL 4427-Rn (SEQ ID NO:7): TGTRCAATCTARTTGCCATATY*CCTGG
POL 4678-R (SEQ ID NO:8): ACTCCY*TGR*CTTTGGGGATTG
TAT 5955-F (SEQ ID NO:9): GCTTAGGCATY*TCCTATGGCAG
TAT 6462-R (SEQ ID NO:10): TGGGGTCTGTK*GGTACACAGG
TAT 6330-Fn (SEQ ID NO:11): CW*GTHTAY*TATGGRGTACCTGTGTGG
Nucleotides indicated by an asterisk (*) indicate degenerate nucleotides known to those skilled in the art.

## Claims

1. A method for diagnosis of a viral infection in a subject, such method comprising separating a cell-free fraction from a urine sample from said subject and detecting the presence of one or more transrenal viral nucleic acids in said cell-free fraction, thereby diagnosing a viral infection in said subject, wherein said transrenal viral nucleic acid is a fragment or fragments smaller than about 1000 base pairs or 1000 nucleotides if denatured.

2. The method according to claim 1, wherein said transrenal viral nucleic acids are DNA.

3. The method according to claim 1, further comprising the step of quantitating said transrenal viral nucleic acids.

4. The method according to claim 3, wherein said quantitating is performed by a means selected from the group consisting of pairing with molecular probes that are specific for those pathogenic agents, hybridization, PCR, nested PCR, semi-nested PCR, SSCP, LCR, and SDA.

5. The method according to claim 1, in which said separation is selected from the group consisting of filtration and centrifugation of said urine sample.

6. The method according to claim 1, in which the length of said transrenal viral nucleic acids is less than that about 500 base pairs.

7. The method according to claim 1, in which said transrenal viral nucleic acids comprise DNA fragments, and the length of said fragments is between about 100 and about 200 bp.

8. The method according to claim 1, further comprising the step of isolation or purification of said transrenal viral nucleic acids.

9. The method according to claim 8, wherein said purification is performed through chemical or physical methods.

10. The method according to claim 8, in which said isolation or purification is performed using a method selected from the group consisting of extraction with organic solvents, filtration, precipitation, absorption on solid matrices having an affinity for the nucleic acids, and chromatography.

11. The method according to claim 10, in which said solid matrices consist of silica-based resins, ion-exchange resins, or hydroxyapatite.

12. The method according to claim 11, in which said solid matrix is a silica-based resin, and said isolation or purification is performed in the presence of a chaotropic agent.

13. The method according to claim 1, further comprising a pretreatment of the urine sample with one or more agents that inhibit the degradation of the nucleic acids.

14. The method according to claim 13, in which said agents are selected from the group consisting of ion-chelating agents, denaturing agents, and ionic detergents.

15. The method according to claim 14, wherein said ion-chelating agents are EDTA; said denaturing agents are guanidine HCl or guanidine isothiocyanate; and said ionic detergents are N-lauryl sarcosine or sodium dodecyl sulfate.

16. The method according to claim 1, wherein said detecting the presence of said transrenal viral nucleic acids is performed through a technique selected from the group consisting of hybridization of the nucleic acids, a cycling probe reaction, PCR, SSCP, LCR, and SDA.

17. The method according to claim 16, in which said PCR is nested or semi-nested PCR.

18. The method according to claim 1, in which said transrenal viral nucleic acid is derived from an RNA or a DNA virus.

19. The method according to claim 18, in which said virus is integrated or episomal.

20. The method according to claim 18, wherein said virus is selected from the group consisting of recombinant and natural HIV-1, HIV-2, variola virus, poliovirus, herpes simplex virus (HSV), Epstein-Barr virus (EBV), hepatitis C virus (HCV), hepatitis B virus (HBV), and adenovirus (AAV).

21. The method according to claim 18, wherein said virus is selected from the group consisting of EBV and HIV-1.

22. The method according to claim 18, wherein said virus is HIV-1.

23. The method according to claim 8, in which said isolated or purified nucleic acid is used for the detection of said transrenal viral nucleic acid.

24. The method according to claim 1, wherein said method is performed *in vitro* and said viral nucleic acid is an HIV nucleic acid.

25. The method according to claim 24, wherein said detection of said transrenal viral nucleic acid is performed through a polymerase chain reaction (PCR).

26. The method according to claim 25, in which said PCR is nested or semi-nested PCR.

27. The method according to claim 25, in which said polymerase chain reaction is performed using one or more primers that are specific for the HIV-1 GAG or POL gene.

28. The method according to claim 27, in which the one or more primers are selected from the group consisting of SEQ ID NOs: 1 to 11.

29. A method for monitoring a viral infection in a subject, such method comprising:
a) separating a first cell-free fraction from a first urine sample from said subject and quantitating the amount of a transrenal viral nucleic acid in said first cell-free fraction;
b) separating a second cell-free fraction from a second urine sample from said subject and quantitating the amount of said transrenal viral nucleic acid in said second cell-free fraction; and
c) comparing the amount of said transrenal viral nucleic acid in said first and said second cell-free fraction,
thereby monitoring said viral infection in said subject,
wherein said transrenal viral nucleic acid is a fragment or fragments smaller than about 1000 base pairs or 1000 nucleotides if denatured.

30. The method according to claim 29, wherein said subject is undergoing treatment with a compound that reduces or inhibits said viral infection.

31. The method according to claim 30, wherein said viral infection is HIV infection and said compound is an anti-viral agent.

32. The method according to claim 29, wherein said quantitating is performed by a means selected from the group consisting of pairing with molecular probes that are specific for those pathogenic agents, hybridization, PCR, nested PCR, SSCP, LCR, and SDA.

33. The method according to claim 29, in which said separation includes centrifugation of said urine sample.

34. The method according to claim 29, in which said transrenal viral nucleic acids comprise DNA fragments, and the length of said fragments is between about 100 and about 200 bp.

35. The method according to claim 29, wherein said subject is at risk of developing a recurring viral infection.

36. A kit for the determination of the presence of a transrenal viral nucleic acid in a urine sample, wherein said transrenal viral nucleic acid is a fragment or fragments smaller than about 1000 base pairs or 1000 nucleotides if denatured, comprising means for isolation or purification of said transrenal viral nucleic acid from a cell-free fraction from a urine ample comprising a solid matrix and an agent that inhibits the degradation of said transrenal viral nucleic acid, and means for determination of the presence of said transrenal viral nucleic acid by hybridization with at least one virus-specific probe.

37. The kit according to claim 36, in which said virus-specific probe comprises a primer for the polymerization chain reaction.

38. The kit according to claim 37, in which said primer is specific for the HIV-1 GAG or POL gene.

## Patentansprüche

1. Verfahren zur Diagnose einer Virusinfektion bei einem Subjekt, wobei ein derartiges Verfahren Abtrennen einer zellfreien Fraktion aus einer Urinprobe von dem Subjekt und Detektieren des Vorliegens einer oder mehrerer transrenaler viraler Nukleinsäuren in der zellfreien Fraktion umfasst, wodurch eine Virusinfektion bei dem Subjekt diagnostiziert wird, wobei die transrenale virale Nukleinsäure ein Fragment oder Fragmente kleiner als etwa 1000 Basenpaare oder 1000 Nukleotide ist, wenn sie denaturiert ist.

2. Verfahren gemäß Anspruch 1, wobei die transrenalen viralen Nukleinsäuren DNA sind.

3. Verfahren gemäß Anspruch 1, das außerdem den Schritt des Quantifizierens der transrenalen viralen Nukleinsäuren umfasst.

4. Verfahren gemäß Anspruch 3, wobei das Quantifizieren durch ein Mittel durchgeführt wird, das aus der Gruppe, bestehend aus Paarung mit Molekularsonden, die spezifisch für solche pathogenen Agenzien sind, Hybridisierung, PCR, verschachtelte PCR, halb-verschachtelte PCR, SSCP, LCR und SDA, ausgewählt ist.

5. Verfahren gemäß Anspruch 1, wobei die Abtrennung aus der Gruppe, bestehend aus Filtration und Zentrifugation der Urinprobe, ausgewählt ist.

6. Verfahren gemäß Anspruch 1, wobei die Länge der transrenalen viralen Nukleinsäuren weniger als etwa 500 Basenpaare ist.

7. Verfahren gemäß Anspruch 1, wobei transrenalen viralen Nukleinsäuren-DNA-Fragmente umfassen und die Länge der Fragmente zwischen etwa 100 und etwa 200 bp ist.

8. Verfahren gemäß Anspruch 1, das außerdem den Schritt der Isolierung oder Reinigung der transrenalen viralen Nukleinsäuren umfasst.

9. Verfahren gemäß Anspruch 8, wobei die Reinigung durch chemische oder physikalische Verfahren durchgeführt wird.

10. Verfahren gemäß Anspruch 8, wobei die Isolierung oder Reinigung unter Verwendung eines Verfahrens, ausgewählt aus der Gruppe, bestehend aus Extraktion mit organischen Lösungsmitteln, Filtration, Präzipitation, Absorption an festen Matrices, die Affinität für die Nukleinsäuren haben, und Chromatographie, ausgewählt wird.

11. Verfahren gemäß Anspruch 10, wobei die festen Matrices aus Silica-basierten Harzen, Ionenaustausch-Harzen oder Hydroxyapatit bestehen.

12. Verfahren gemäß Anspruch 11, indem die feste Matrix ein Silica-basiertes Harz ist und die Isolierung oder Reinigung in Gegenwart eines chaotropen Mittels durchgeführt wird.

13. Verfahren gemäß Anspruch 1, das außerdem eine Vorbehandlung der Urinprobe mit einem oder mehreren Mitteln, das/die den Abbau der Nukleinsäuren inhibiert/inhibieren, umfasst.

14. Verfahren gemäß Anspruch 13, wobei die Mittel aus der Gruppe, bestehend aus Ionen-chelatisierenden Mitteln, denaturierenden Mitteln und ionischen Detergenzien, ausgewählt werden.

15. Verfahren gemäß Anspruch 14, wobei die Ionen-chelatisierenden Mittel EDTA sind, die Denaturierungsmittel Guanidin-HCl oder Guanidinisothiocyanat sind und die ionischen Detergenzien N-Laurylsarcosin oder Natriumdodecylsulfat sind.

16. Verfahren gemäß Anspruch 1, wobei das Detektieren des Vorliegens der transrenalen viralen Nukleinsäuren durch eine Technik durchgeführt wird, die aus der Gruppe, bestehend aus Hybridisierung der Nukleinsäuren, einer Cyclisierungssondenreaktion, PCR, SSCP, LCR und SDA, ausgewählt wird.

17. Verfahren gemäß Anspruch 16, wobei die PCR eine verschachtelte oder halb-verschachtelte PCR ist.

18. Verfahren gemäß Anspruch 1, wobei die transrenale virale Nukleinsäure von einer RNA oder einem DNA-Virus abgeleitet ist.

19. Verfahren gemäß Anspruch 18, wobei das Virus integriert oder episomal ist.

20. Verfahren gemäß Anspruch 18, wobei das Virus aus der Gruppe, bestehend aus rekombinantem und natürlichem HIV-1, HIV-2, Variola-Virus, Polio-Virus, Herpes-Simplex-Virus (HSV), Epstein-Barr-Virus (EBV), Hepatitis-C-Virus (HCV), Hepatitis-B-Virus (HBV) und Adenovirus (AAV), ausgwählt wird.

21. Verfahren gemäß Anspruch 18, wobei das Virus aus der Gruppe, bestehend aus EBV und HIV-1, ausgewählt wird.

22. Verfahren gemäß Anspruch 18, wobei das Virus HIV-1 ist.

23. Verfahren gemäß Anspruch 8, wobei die isolierte oder gereinigte Nukleinsäure für die Detektion der transrenalen, viralen Nukleinsäure verwendet wird.

24. Verfahren gemäß Anspruch 1, wobei das Verfahren *in vitro* durchgeführt wird und die virale Nukleinsäure eine HIV-Nukleinsäure ist.

25. Verfahren gemäß Anspruch 24, wobei die Detektion der transrenalen viralen Nukleinsäure durch eine Polymerasekettenreaktion (PCR) durchgeführt wird.

26. Verfahren gemäß Anspruch 25, wobei die PCR verschachtelte oder halb-verschachtelte PCR ist.

27. Verfahren gemäß Anspruch 25, wobei die Polymerasekettenreaktion unter Verwendung eines oder mehrerer Primer durchgeführt wird, die für das HIV-1-GAG- oder -POL-Gen spezifisch sind.

28. Verfahren gemäß Anspruch 27, wobei der eine oder die mehreren Primer aus der Gruppe, bestehend aus SEQ-ID-NOs 1 bis 11, ausgewählt werden.

29. Verfahren zur Überwachung einer Virusinfektion bei einem Subjekt, wobei ein derartiges Verfahren umfasst:
(a) Abtrennen einer ersten zellfreien Fraktion aus einer ersten Urinprobe von dem Subjekt und Quantifizieren der Menge einer transrenalen viralen Nukleinsäure in der ersten zellfreien Fraktion;
(b) Abtrennen einer zweiten zellfreien Fraktion aus einer zweiten Urinprobe von dem Subjekt und Quantifizieren der Menge der transrenalen viralen Nukleinsäure in der zweiten zellfreien Fraktion und
(c) Vergleichen der Menge der transrenalen viralen Nukleinsäure in der ersten und der zweiten zellfreien Fraktion,
wodurch die Virusinfektion bei dem Subjekt überwacht wird, wobei die transrenale virale Nukleinsäure ein Fragment oder Fragmente kleiner als etwa 1000 Basenpaare oder 1000 Nukleotide, wenn sie denaturiert ist, ist.

30. Verfahren gemäß Anspruch 29, wobei das Subjekt sich einer Behandlung mit einer Verbindung, die die Virusinfektion reduziert oder inhibiert, unterzieht.

31. Verfahren gemäß Anspruch 30, wobei die Virusinfektion HIV-Infektion ist und die Verbindung ein antivirales Mittel ist.

32. Verfahren gemäß Anspruch 29, wobei das Quantifizieren durch ein Mittel durchgeführt wird, das aus der Gruppe, bestehend aus Paarung mit Molekularsonden, die für solche pathogenen Agenzien spezifisch sind, Hybridisierung, PCR, verschachtelte PCR, SSCP, LCR und SDA, ausgewählt wird.

33. Verfahren gemäß Anspruch 29, wobei die Abtrennung Zentrifugation der Urinprobe umfasst.

34. Verfahren gemäß Anspruch 29, wobei die transrenalen viralen Nukleinsäuren DNA-Fragmente umfassen und die Länge der Fragmente zwischen etwa 100 und etwa 200 bp ist.

35. Verfahren gemäß Anspruch 29, wobei das Subjekt ein Risiko zur Entwicklung einer wiederkehrenden Virusinfektion hat.

36. Kit für die Bestimmung des Vorliegens einer transrenalen viralen Nukleinsäure in einer Urinprobe, wobei die transrenale virale Nukleinsäure ein Fragment oder Fragmente kleiner als etwa 1000 Basenpaare oder 1000 Nukleotide, wenn sie denaturiert ist, ist, umfassend Mittel zur Isolierung oder Reinigung der transrenalen viralen Nukleinsäure aus einer zellfreien Fraktion aus einer Urinprobe, umfassend eine feste Matrix und ein Mittel, das den Abbau der transrenalen Nukleinsäure inhibiert, und Mittel zur Bestimmung des Vorliegens der transrenalen viralen Nukleinsäure durch Hybridisierung mit wenigstens einer Virus-spezifischen Sonde.

37. Kit gemäß Anspruch 36, wobei die Virus-spezifische Sonde einen Primer für die Polymerisationskettenreaktion umfasst.

38. Kit gemäß Anspruch 37, wobei der Primer für das HIV-1-GAG- oder -POL-Gen spezifisch ist.

## Revendications

1. Procédé de diagnostic d'une infection virale chez un sujet, lequel procédé comporte le fait de séparer une fraction sans cellules d'un échantillon d'urine provenant dudit sujet, et le fait de détecter la présence d'un ou de plusieurs acide(s) nucléique(s) viral(aux) transrénal(aux) dans ladite fraction sans cellules, ce qui permet de diagnostiquer une infection virale chez ledit sujet, étant entendu que ledit ou lesdits acide(s) nucléique(s) viral(aux) transrénal(aux) est ou sont un fragment ou des fragments de longueur inférieure à environ 1000 paires de bases, ou 1000 nucléotides s'il est ou s'ils sont dénaturé(s).

2. Procédé conforme à la revendication 1, dans lequel lesdits acides nucléiques viraux transrénaux sont des ADN.

3. Procédé conforme à la revendication 1, qui comporte en outre une étape de mesure de la quantité desdits acides nucléiques viraux transrénaux.

4. Procédé conforme à la revendication 3, dans lequel ladite mesure de quantité est effectuée selon une technique choisie dans l'ensemble formé par les suivantes : appariement avec des sondes moléculaires spécifiques pour ces agents pathogènes, hybridation, PCR, PCR nichée, PCR semi-nichée, SSCP, LCR et SDA.

5. Procédé conforme à la revendication 1, dans lequel ladite opération de séparation est choisie parmi une filtration et une centrifugation dudit échantillon d'urine.

6. Procédé conforme à la revendication 1, dans lequel la longueur desdits acides nucléiques viraux transrénaux est inférieure à environ 500 paires de bases.

7. Procédé conforme à la revendication 1, dans lequel lesdits acides nucléiques viraux transrénaux comprennent des fragments d'ADN, et la longueur de ces fragments vaut d'environ 100 à environ 200 paires de bases.

8. Procédé conforme à la revendication 1, qui comporte en outre une étape d'isolement ou de purification desdits acides nucléiques viraux transrénaux.

9. Procédé conforme à la revendication 8, dans lequel ladite purification est opérée par des procédés chimiques ou physiques.

10. Procédé conforme à la revendication 8, dans lequel ledit isolement ou ladite purification est opéré(e) au moyen d'un procédé choisi dans l'ensemble constitué par les suivants : extraction par solvants organiques, filtration, précipitation, absorption sur des matrices solides possédant une affinité pour les acides nucléiques, et chromatographie.

11. Procédé conforme à la revendication 10, dans lequel lesdites matrices solides consistent en des résines à base de silice, des résines échangeuses d'ions ou de l'hydroxy-apatite.

12. Procédé conforme à la revendication 11, dans lequel ladite matrice solide est une résine à base de silice et ledit isolement ou ladite purification est opéré(e) en présence d'un agent chaotropique.

13. Procédé conforme à la revendication 1, qui comporte en outre un prétraitement de l'échantillon d'urine avec un ou plusieurs agent(s) qui inhibe(nt) la dégradation des acides nucléiques.

14. Procédé conforme à la revendication 13, dans lequel lesdits agents sont choisis dans l'ensemble constitué par les agents chélateurs d'ions, les agents dénaturants et les détergents ioniques.

15. Procédé conforme à la revendication 14, dans lequel lesdits agents chélateurs d'ions sont de l'EDTA, lesdits agents dénaturants sont du chlorhydrate de guanidine ou de l'isothiocyanate de guanidine, et lesdits détergents ioniques sont de la N-lauryl-sarcosine ou du dodécyl-sulfate de sodium.

16. Procédé conforme à la revendication 1, dans lequel ladite détection de la présence desdits acides nucléiques viraux transrénaux est effectuée selon une technique choisie dans l'ensemble formé par les suivantes : hybridation d'acides nucléiques, réaction de sondes par cyclage, PCR, SSCP, LCR et SDA.

17. Procédé conforme à la revendication 16, dans lequel ladite PCR est une PCR nichée ou une PCR semi-nichée.

18. Procédé conforme à la revendication 1, dans lequel ledit acide nucléique viral transrénal dérive d'un virus à ARN ou d'un virus à ADN.

19. Procédé conforme à la revendication 18, dans lequel ledit virus est un virus intégré ou un épisome viral.

20. Procédé conforme à la revendication 18, dans lequel ledit virus est choisi dans l'ensemble constitué par les suivants, recombinés ou naturels : VIH-1, VIH-2, virus de variole, poliovirus, virus d'herpès simplex (HSV), virus d'Epstein-Barr (EBV), virus d'hépatite C (VHC), virus d'hépatite B (VHB), et adénovirus (AAV).

21. Procédé conforme à la revendication 18, dans lequel ledit virus est choisi parmi les virus EBV et VIH-1.

22. Procédé conforme à la revendication 18, dans lequel ledit virus est le virus VIH-1.

23. Procédé conforme à la revendication 8, dans lequel ledit acide nucléique isolé ou purifié est utilisé pour la détection dudit acide nucléique viral transrénal.

24. Procédé conforme à la revendication 1, lequel procédé est mis en oeuvre *in vitro* et dans lequel procédé ledit acide nucléique viral est un acide nucléique de VIH.

25. Procédé conforme à la revendication 24, dans lequel ladite détection dudit acide nucléique viral transrénal est effectuée par PCR (amplification en chaîne par polymérase).

26. Procédé conforme à la revendication 25, dans lequel ladite PCR est une PCR nichée ou une PCR semi-nichée.

27. Procédé conforme à la revendication 25, dans lequel ladite amplification en chaîne par polymérase est réalisée au moyen d'une ou de plusieurs amorces, spécifique(s) pour le gène *gag* ou *pol* de VIH-1.

28. Procédé conforme à la revendication 27, dans lequel l'amorce ou les amorces est ou sont choisie(s) dans l'ensemble constitué par les Séquences N° 1 à 11.

29. Procédé de surveillance d'une infection virale chez un sujet, lequel procédé comporte les étapes suivantes :
a) séparer une première fraction sans cellules d'un premier échantillon d'urine provenant dudit sujet, et mesurer la quantité d'un acide nucléique viral transrénal dans cette première fraction sans cellules,
b) séparer une deuxième fraction sans cellules d'un deuxième échantillon d'urine provenant dudit sujet, et mesurer la quantité dudit acide nucléique viral transrénal dans cette deuxième fraction sans cellules,
c) et comparer les quantités dudit acide nucléique viral transrénal dans lesdites première et deuxième fractions sans cellules,
ce qui permet de surveiller ladite infection virale chez ledit sujet, étant entendu que ledit acide nucléique viral transrénal est un fragment ou des fragments de longueur inférieure à environ 1000 paires de bases, ou 1000 nucléotides s'il est ou s'ils sont dénaturé(s).

30. Procédé conforme à la revendication 29, dans lequel ledit sujet est en cours de traitement avec un composé qui réduit ou inhibe ladite infection virale.

31. Procédé conforme à la revendication 30, dans lequel ladite infection virale est une infection par VIH et ledit composé est un agent anti-viral.

32. Procédé conforme à la revendication 29, dans lequel ladite mesure de quantité est effectuée selon une technique choisie dans l'ensemble formé par les suivantes : appariement avec des sondes moléculaires spécifiques pour ces agents pathogènes, hybridation, PCR, PCR nichée, SSCP, LCR et SDA.

33. Procédé conforme à la revendication 29, dans lequel ladite séparation inclut une centrifugation dudit échantillon d'urine.

34. Procédé conforme à la revendication 29, dans lequel lesdits acides nucléiques viraux transrénaux comprennent des fragments d'ADN, et la longueur de ces fragments vaut d'environ 100 à environ 200 paires de bases.

35. Procédé conforme à la revendication 29, dans lequel ledit sujet présente un risque de développer une infection virale ou de faire une rechute d'infection virale.

36. Trousse servant à déterminer la présence d'un acide nucléique viral transrénal dans un échantillon d'urine, lequel acide nucléique viral transrénal est un fragment ou des fragments de longueur inférieure à environ 1000 paires de bases, ou 1000 nucléotides s'il est ou s'ils sont dénaturé(s), laquelle trousse comprend des moyens qui servent à isoler ou à purifier ledit acide nucléique viral transrénal à partir d'une fraction sans cellules d'un échantillon d'urine et qui comprennent une matrice solide et un agent qui inhibe la dégradation dudit acide nucléique viral transrénal, et des moyens qui servent à déterminer la présence dudit acide nucléique viral transrénal par hybridation avec au moins une sonde spécifique d'un virus.

37. Trousse conforme à la revendication 36, dans laquelle ladite sonde spécifique d'un virus comporte une amorce pour PCR.

38. Trousse conforme à la revendication 37, dans laquelle ladite amorce est spécifique pour le gène *gag* ou *pol* de VIH-1.
